# EUROPEAN PATENT APPLICATION

(11) **EP 4 295 782 A1**
(43) Date of publication of application: **27.12.2023**
(21) Application number: 21926715.0
(22) Date of filing: 15.11.2021
(51) Int. Cl.: A61B 10/00, A61B 5/08, A61B 5/11, A61B 5/113

(54) **EVALUATION DEVICE, EVALUATION SYSTEM, EVALUATION METHOD, EVALUATION PROGRAM, AND RECORDING MEDIUM**

(30) Priority: 18.02.2021 JP 2021023870
(71) Applicant: Osaka University, Suita-shi Osaka 565-0871 (JP); Heartlab, Inc., Kobe-shi, Hyogo 650-0047 (JP)
(72) Inventor: MIYAGAWA, Shigeru, Suita-shi, Osaka 565-0871 (JP); SAWA, Yoshiki, Suita-shi, Osaka 565-0871 (JP); ASANOI, Hidetsugu, Suita-shi, Osaka 565-0871 (JP); IKEGAWA, Sunao, Kobe-shi, Hyogo 650-0047 (JP)
(74) Representative: Wynne-Jones IP Limited
(86) International application number: PCT/JP2021/041823
(87) International publication number: WO 2022/176287

(57) **Abstract**

The present invention relates to an evaluation device 4 for evaluating the severity of pneumonia in a target patient, comprising: an acquisition unit 42 for acquiring a respiratory waveform of the target patient; a calculation unit 43 for calculating a value of an index indicating instability of a respiratory cycle or a respiratory frequency from the respiratory waveform; and an evaluation unit 44 for evaluating the severity based on the calculated value.

## Description

### Technical Field

The present invention relates to a technique for evaluating the severity of pneumonia, and particularly to a technique for evaluating the severity of pneumonia caused by COVID-19.

### Background Art

COVID-19 has propagated throughout the world, with the cumulative number of patients exceeding 100 million and the number of deaths reaching 2.1 million in late January 2021. In Japan as well, there were 7,882 new patients in one day on January 8, 2021, and not all of the patients could be hospitalized in medical institutions. In other words, most COVID-19 patients have to stay at accommodation facilities or at home for recovery, and only patients who have become so severe as to require advanced treatment during convalescence have to be hospitalized.

With respect to the health observation of COVID-19 patients who stay at accommodation facilities or at home for recovery, Japan's Ministry of Health, Labour and Welfare has established a system in which health states are periodically grasped using information communication devices such as telephones, mainly by health centers of prefectures; and patients can promptly have medical examinations at appropriate medical institutions when their symptoms worsen (page 4 of Non-patent Literature 1 below). In addition, the Ministry of Health and Labour delivers pulse oximeters to home care patients, and promotes the health monitoring of the home care patients by checking their oxygen saturation level (Non-patent Literature 2 below).

### Citation List

### Non-patent Literature

NPL 1: "Points to note for home care patients with mild symptoms of novel Coronavirus infection," the Ministry of Health, Labour and Welfare, Novel Coronavirus Infection Countermeasure Promotion Division, May 1, 2020 (revised on August 7, 2020), the internet (https://www.mhlw.go.jp/content/000657891.pdf)
NPL 2: "Utilization of Pulse Oximeter for Health Observation in Home Recovery," the Ministry of Health, Labour and Welfare, Novel Coronavirus Infection Countermeasure Promotion Division, January 28, 2021, the internet (URL: https://www.mhlw.go.jp/content/000732500.pdf).

### Summary of Invention

### Technical Problem

COVID-19 is characterized in that it causes complications such as respiratory failure due to pneumonia, cardiovascular diseases such as arrhythmia and cardiac disorder, and thromboembolism such as pulmonary embolism and acute stroke; and rapidly becomes severe and causes death. COVID-19 patients who have stayed at accommodation facilities or at home for recovery check their subjective symptoms, body temperature, and oxygen saturation, and are triaged due to the necessity of inpatient treatment based on the results. However, subjective symptoms and body temperature have low specificity for pneumonia, and are thus less likely to be factors for determining triage. Although oxygen saturation is important information for triage, the action of measuring the oxygen saturation by the patient themselves in a poor physical condition and reporting the data to an administrator of a health center or the like is necessary, which is a burden on the patient. Moreover, even if these are self-checked, unfortunately, there are successively cases in which patients who stay at accommodation facilities or at home for recovery die.

In order to prevent the death of patients who stay at accommodation facilities or at home for recovery, there is a need for a system that is capable of remotely monitoring the medical condition of a large number of patients without requiring the patient's own action, and easily determining the necessity of hospitalization from the information.

The present invention has been made to solve the above problems, and an object of the present invention is to easily evaluate the severity of pneumonia.

### Solution to Problem

In order to solve the above problems, the present invention includes the following aspects.
1. An evaluation device for evaluating the severity of pneumonia in a target patient, comprising:
   an acquisition unit for acquiring a respiratory waveform of the target patient;
   a calculation unit for calculating a value of an index indicating instability of a respiratory cycle or a respiratory frequency from the respiratory waveform; and
   an evaluation unit for evaluating the severity based on the calculated value.
2. The evaluation device according to Item 1, wherein the calculation unit calculates RST, which is a reciprocal of a standard deviation of the respiratory frequency, as the value of the index.
3. The evaluation device according to Item 2, wherein the evaluation unit evaluates that the severity is high when the RST is less than a threshold value.
4. The evaluation device according to any one of Items 1 to 3, wherein the pneumonia is caused by COVID-19.
5. An evaluation system comprising:
   a detection device for detecting a signal comprising a respiratory waveform of the target patient; and
   the evaluation device according to any one of Items 1 to 4.
6. The evaluation system according to Item 5, wherein the detection device comprises a sheet sensor provided below the target patient on a bed, and the sheet sensor detects a generated body pressure signal as the signal.
7. An evaluation method for evaluating the severity of pneumonia in a target patient, comprising:
   an acquisition step of acquiring a respiratory waveform of the target patient;
   a calculation step of calculating a value of an index indicating instability of a respiratory cycle or a respiratory frequency from the respiratory waveform; and
   an evaluation step of evaluating the severity based on the calculated value.
8. An evaluation program for causing a computer to operate as each unit of the evaluation device according to any one of Items 1 to 4.
9. A computer-readable recording medium in which the evaluation program according to Item 8 is recorded.

### Advantageous Effects of Invention

According to the present invention, the severity of pneumonia is evaluated based on the value of an index indicating instability of a respiratory cycle or a respiratory frequency such as RST. Since the index can be easily monitored from a remote target patient, the severity of pneumonia can be easily evaluated.

### Brief Description of Drawings

Fig. 1 shows a block diagram illustrating a schematic configuration of an evaluation system according to an embodiment of the present invention.
Fig. 2 shows a schematic diagram illustrating an installation example of a detection device and a relay terminal.
Fig. 3 shows a flowchart illustrating a processing procedure of an evaluation method for evaluating the severity of pneumonia of a target patient using the evaluation system according to the embodiment.
Fig. 4 shows a box plot comparing RSTs of patients with "moderate disease II/severe disease" and patients with "mild/moderate disease I" as shown in the "COVID-19 Medical Guide" published by the Ministry of Health, Labour and Welfare.
Fig. 5 shows a box plot comparing RSTs of patients with a score of "Medium or High" and patients with a score of "Low or Low-medium" as indicated by the National Early Warning Score (NEWS).
Fig. 6 shows a box plot comparing RSTs of patients with and without pneumonia images on CT.
Fig. 7 shows an ROC curve for the severity of COVID-19 patients.

### Description of Embodiments

Hereinafter, embodiments of the present invention will be described with reference to the accompanying drawings. The present invention is not limited to the following embodiments.

### System Configuration

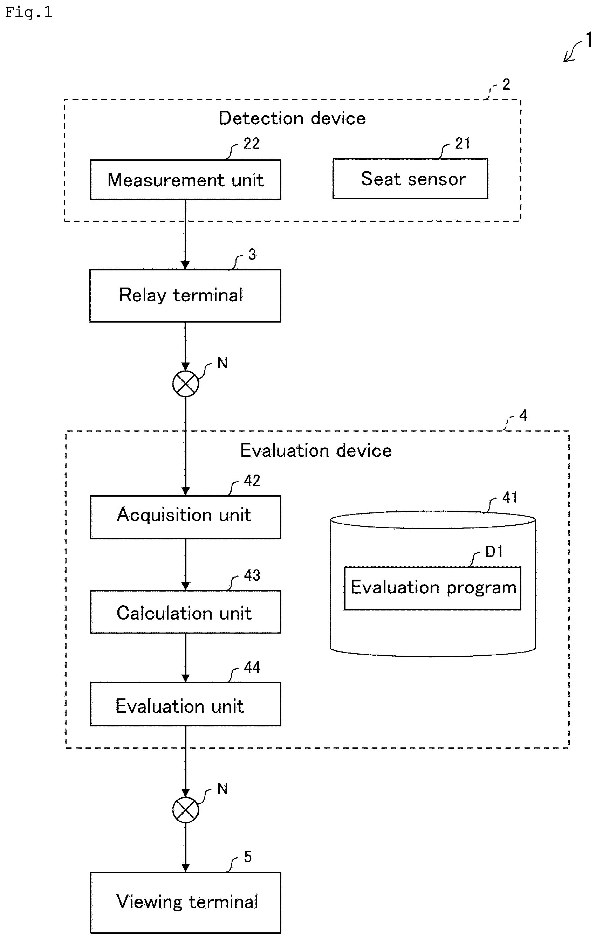

Fig. 1 is a block diagram illustrating a schematic configuration of an evaluation system 1 according to an embodiment of the present invention. The evaluation system 1 is a system for evaluating the severity of the pneumonia of a target patient, and includes a detection device 2, a relay terminal 3, an evaluation device 4, and a viewing terminal 5.

In this embodiment, the target patient is a person who is determined to be positive for COVID-19 and stays at home or an accommodation facility for recovery. The detection device 2 and the relay terminal 3 are provided in a home or accommodation facility where the target patient stays. In addition, the evaluation device 4 is provided in the cloud, and the viewing terminal 5 is provided in an institution (health center, hospital, etc.) that performs health monitoring of a target patient. The evaluation device 4 and the viewing terminal 5 may be configured as a single device.

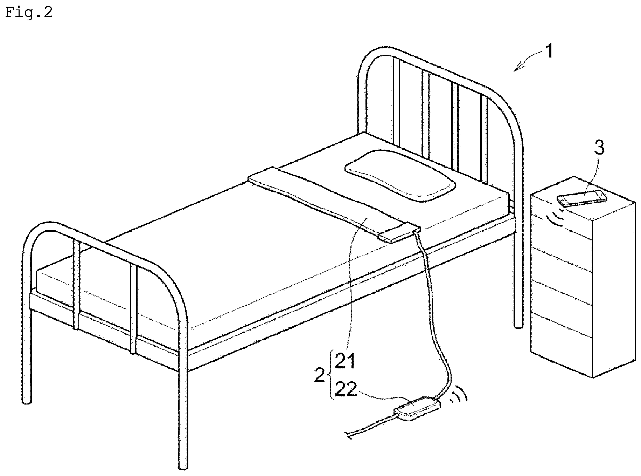

Fig. 2 is a schematic diagram illustrating an installation example of the detection device 2 and the relay terminal 3. The detection device 2 is a device that detects a signal including a respiratory waveform of a target patient, and includes a sheet sensor 21 and a measurement unit 22.

The sheet sensor 21 is a piezoelectric body motion sensor formed of a thin and soft band-shaped piezoelectric rubber, and is provided, for example, on a bed used by a target patient. In Fig. 2, the sheets and the duvet laid on the sheet sensor 21 are not illustrated. While the target patient is in the bed, pressure is applied to the sheet sensor 21, and the sheet sensor 21 generates an analog body pressure signal by the piezoelectric effect. The body pressure signal includes, in addition to the respiratory waveform, a waveform due to noise and body motion other than respiration.

The measurement unit 22 is connected to the sheet sensor 21, and the seat sensor 21 performs AD conversion of a generated body pressure signal. The measurement unit 22 has a function of communicating using the relay terminal 3 and Bluetooth (registered trademark), and transmits a body pressure signal to the relay terminal 3.

The relay terminal 3 is composed of a smartphone, and transmits the body pressure signal received from the measurement unit 22 to the evaluation device 4 in the cloud via the internet N. If the measurement unit 22 can be connected to the internet N, the relay terminal 3 may be omitted, and the body pressure signal may be directly transmitted from the measurement unit 22 to the evaluation device 4.

The evaluation device 4 shown in Fig. 1 can be configured as a server device. The evaluation device 4 includes a memory unit 41, an acquisition unit 42, a calculation unit 43, and an evaluation unit 44.

The memory unit 41 can be formed of, for example, an HDD or an SSD. The memory unit 41 stores various data such as an evaluation program D1.

Each unit of the acquisition unit 42, the calculation unit 43, and the evaluation unit 44 may be realized in a hardware manner using a logic circuit or the like, or may be realized in a software manner using a CPU or the like. In a case where the respective units are realized by software, the respective units can be realized by a CPU or the like of the evaluation device 4 reading the evaluation program D1 into a main storage device and executing the evaluation program D1. The evaluation program D1 may be downloaded to the evaluation device 4 via a communication network such as the internet N, or may be installed in the evaluation device 4 via a computer-readable non-transitory recording medium such as a CD-R in which the evaluation program D1 is recorded.

The acquisition unit 42 has a function of acquiring a respiratory waveform of a target patient. In this embodiment, the acquisition unit 42 extracts a respiratory waveform by removing a waveform due to body motion other than respiration and noise from a body pressure signal received from the detection device 2 via the relay terminal 3.

The calculation unit 43 has a function of calculating the value of the index indicating the instability of the respiratory cycle or the respiratory frequency from the respiratory waveform obtained by the acquisition unit 42. In this embodiment, the calculation unit 43 performs frequency analysis (maximum entropy method) on the respiratory waveform to calculate the respiratory stability time (RST), which is the reciprocal of the standard deviation of the respiratory frequency, as the value of the index. Specifically, the RST is calculated as a reciprocal of a standard deviation of a frequency of 5% or more of a maximum value of a respiratory frequency component, which is obtained by extracting a band of a respiratory cycle from a respiratory waveform.
RST=1/A

### A: Standard deviation of frequency of 5% or more of a maximum value of a respiratory frequency component

The evaluation unit 44 has a function of evaluating the severity of pneumonia of the target patient based on the value calculated by the calculation unit 43. In this embodiment, the evaluation unit 44 evaluates that the severity is high when the RST is less than a threshold value. When the target patient is a person who is positive for COVID-19, the threshold value is preferably 20 to 30 seconds, and more preferably 26 seconds.

The evaluation result by the evaluation unit 44 is stored in the storage unit 41 and is transmitted to the viewing terminal 5 via the internet N. In the viewing terminal 5, the RSTs of the target patients are displayed in a list, and the numerical values of the target patients evaluated as having high severity are displayed in a mode (different colors, highlighting, etc.) different from that of the other patients. Accordingly, the user (such as the administrator of the health center) of the viewing terminal 5 can easily identify a target patient who needs to be hospitalized.

### Procedure

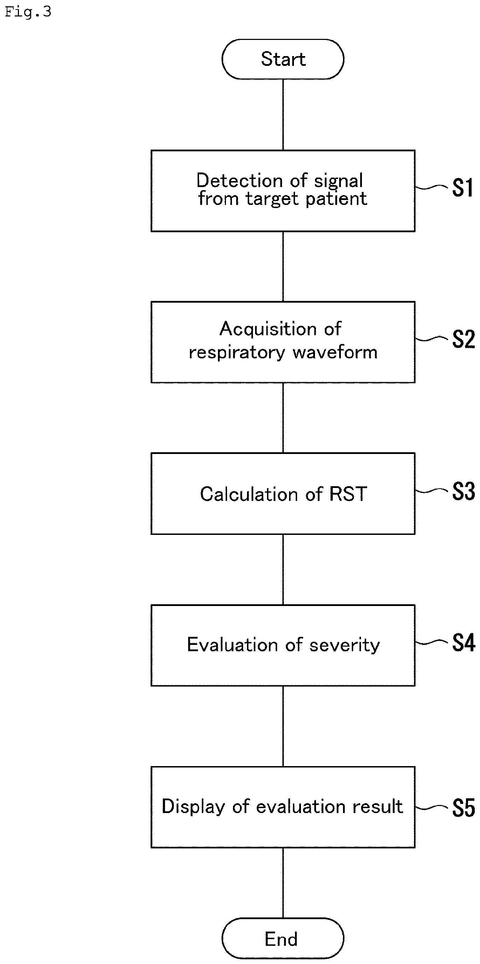

Fig. 3 is a flowchart illustrating a procedure of an evaluation method for evaluating the severity of pneumonia of a target patient using the evaluation system 1 according to this embodiment.

In step S1, the sheet sensor 21 of the detection device 2 detects the body pressure signal of the target patient in the bed. In step S2 (acquisition step), the acquisition unit 42 of the evaluation device 4 acquires the respiratory waveform of the target patient from the body pressure signal received from the detection device 2. In step S3 (calculation step), the calculation unit 43 of the evaluation device 4 calculates the RST from the respiratory waveform acquired by the acquisition unit 42. In step S4 (evaluation step), the evaluation unit 44 of the evaluation device 4 evaluates the severity of pneumonia of the target patient based on the value calculated by the calculation unit 43. In step S5, the viewing terminal 5 displays the evaluation result by the evaluation unit 44.

Note that the operation subject of each step is not limited to the above.

### Effect

As described above, in this embodiment, the severity of pneumonia is evaluated based on the RST. The RST is obtained by automatically extracting and analyzing a respiratory waveform from a body pressure signal generated by the sheet sensor 21 provided under the target patient who is in the bed. Specifically, the RST is obtained in a non-invasive, unrestrained environment for the patient without requiring special action of the patient themselves, as in the case of a pulse oximeter. In addition, since it is not necessary for the administrator or the like to face the target patient, it is possible to monitor the RST from a remote location easily, inexpensively, and continuously every day even for a patient isolated at home or at an accommodation facility due to an infectious disease such as COVID-19. Further, when the RST is less than a certain threshold value, it can be determined that the condition has deteriorated, such as in the onset of pneumonia.

As described above, when the evaluation system according to this embodiment is used, it is possible to detect a patient who needs hospitalization treatment based on the RST. As a result, a patient who needs to be hospitalized can be admitted to the hospital in a timely manner; accordingly, it is not only possible to reduce the number of deaths of patients who stay at accommodation facilities or at home for recovery, but to also give the patients a sense of security that they are being monitored remotely.

In addition, since a small number of employees of a health center or the like can collectively manage a large number of patients who stay at accommodation facilities or at home for recovery, the effective use of medical resources also becomes possible. For the patient, it is possible to reduce their mental and physical burden, as well as their economic burden, by the early detection of exacerbation.

### Principle

The RST is an index created by the inventors of the present application to quantitatively evaluate a change in breathing pattern.

There are four extension receptors in the bronchi and interstitium of the lung. An increase in pulmonary arterial pressure or interstitial edema due to heart failure stimulates these biosensors and destabilizes breathing. In addition, delayed circulation due to heart failure and enhanced central chemoreflex also destabilize respiration and induce periodic respiration. The RST that can be calculated from the frequency analysis of the respiratory waveform is excellent in that these unstable breaths can be quantified by a single index.

In the case of a pneumonia patient such as pneumonia caused by COVID-19 or aspiration pneumonia, it seems that the lung extension receptors are stimulated by inflammation of the lung parenchyma, and thus, respiration becomes unstable. Therefore, there is considered to be a high possibility that aggravation of pneumonia can be detected at an early stage by remotely monitoring the RST in the evaluation system according to this embodiment. In addition, the evaluation system according to this embodiment enables preemptive medical treatment that places importance on individuality, leading to an improvement in the QOL of a patient, such as the avoidance of early treatment and hospitalization, and a medical economic effect.

### Additional Remarks

Although embodiments of the present invention have been described above, the present invention is not limited to the above-described embodiments, and various modifications can be made without departing from the gist of the present invention.

In the above embodiment, the respiratory waveform is acquired by removing the waveform due to the body motion other than the respiration or the noise from the body pressure signal detected by the sheet sensor. However, the present invention is not limited thereto; for example, the respiratory waveform may be acquired by a respiratory airflow sensor attached to the skin surface near the nasal cavity of the target patient. In addition, a depth sensor, a radar Doppler, an ultrasonic Doppler, or the like may be used as the detection device of the respiratory signal. However, since the burden on the patient themselves increases, the above embodiment is preferable from the viewpoint of convenience.

In the above embodiment, the RST is used as an index for evaluating the severity of pneumonia. However, the RST is not particularly limited as long as it is an index indicating instability of a respiratory cycle or a respiratory frequency equivalent to the RST.

Further, the severity of pneumonia may be evaluated in consideration of the respiratory rate and the heart rate in addition to the RST. In this case, the calculation unit 43 shown in Fig. 1 calculates the RST, the respiratory rate, and the heart rate from the respiratory waveform, and the evaluation unit 44 evaluates the severity of pneumonia based on the RST, the respiratory rate, and the heart rate. Accordingly, the accuracy of the evaluation can be further improved.

### Examples

Hereinafter, examples of the present invention will be described. The present invention is not limited to the following examples.

The inventors of the present application remotely monitored the RST overnight from the body pressure signal of the sheet sensor 21 using 40 patients throughout Japan (32 COVID-19 patients and 8 pneumonia patients) from July 31, 2020 to November 19, 2020. As the sheet sensor 21, a body motion sensor produced by Sumitomo Riko Co., Ltd. was used.

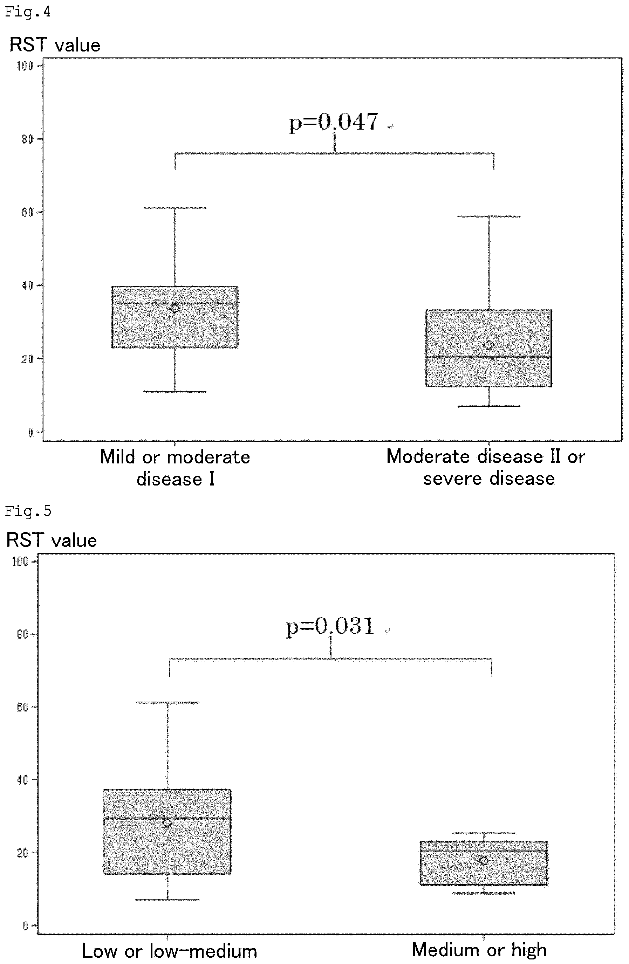

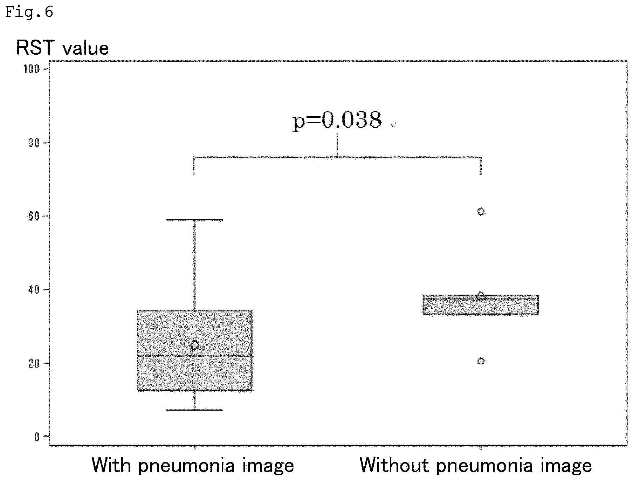

As a result, the RST values of the patients with "moderate disease II/severe disease" as indicated in the "COVID-19 Medical Care Guide" published by the Ministry of Health, Labour and Welfare were significantly lower than those of the patients with "mild/moderate disease I " (Fig. 4). Similarly, patients with scores of "Medium or High" as indicated by the National Early Warning Score (NEWS) had significantly lower RST values than patients with scores of "Low or Low-medium" (Fig. 5). In addition, patients with a pneumonia image (with infiltration) on CT had significantly lower RST values than patients without a pneumonia image (Fig. 6).

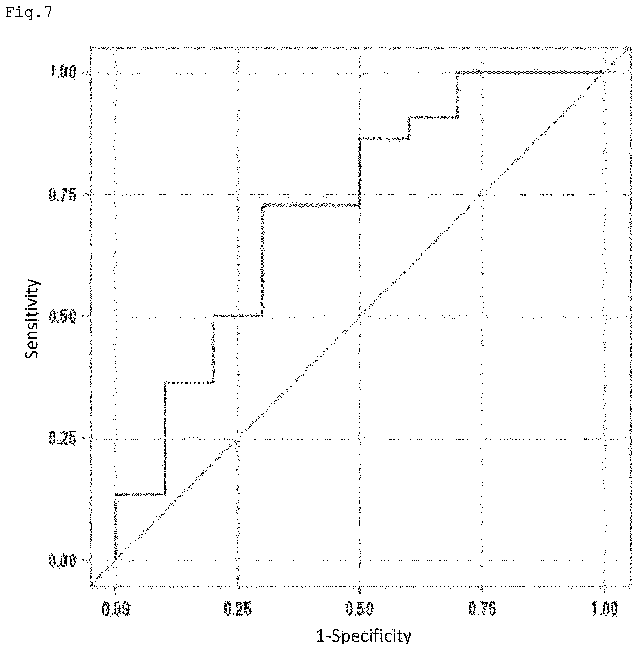

Only COVID-19 patients were examined. The RST during hospitalization at which the highest sensitivity and specificity are attained for the presence or absence of future severe disease was examined; the cut-off threshold was 26.3 seconds. Fig. 7 shows the ROC curve at its optimum cut-off value. The sensitivity was 0.727, the specificity was 0.700, and the area under the curve was 0.7227.

These results suggest that the RST threshold, which is the criterion for evaluating whether a patient falls into the moderate II or severe category, is around 26 seconds. Accordingly, if the RST falls below 20 to 30 seconds, it can be determined that the pneumonia is severe enough to require hospitalization.

### Industrial Applicability

The present invention is particularly suitable for, but not limited to, monitoring isolated COVID-19 positive individuals. For example, the present invention can be applied in geriatric healthcare facilities to monitor the RSTs of residents, enabling early detection of aspiration pneumonia and other conditions.

### Explanation of Reference Numerals

1 Evaluation system
2 Detection device
21 Seat sensor
22 Measurement unit
3 Relay terminal
4 Evaluation device
41 Memory unit
42 Acquisition unit
43 Calculation unit
44 Evaluation unit
5 Viewing terminal
D1 Evaluation program

## Claims

1. An evaluation device for evaluating the severity of pneumonia in a target patient, comprising:
an acquisition unit for acquiring a respiratory waveform of the target patient;
a calculation unit for calculating a value of an index indicating instability of a respiratory cycle or a respiratory frequency from the respiratory waveform; and
an evaluation unit for evaluating the severity based on the calculated value.

2. The evaluation device according to claim 1, wherein the calculation unit calculates RST, which is a reciprocal of a standard deviation of the respiratory frequency, as the value of the index.

3. The evaluation device according to claim 2, wherein the evaluation unit evaluates that the severity is high when the RST is less than a threshold value.

4. The evaluation device according to any one of claims 1 to 3, wherein the pneumonia is caused by COVID-19.

5. An evaluation system comprising:
a detection device for detecting a signal comprising a respiratory waveform of the target patient; and
the evaluation device according to any one of claims 1 to 4.

6. The evaluation system according to claim 5, wherein the detection device comprises a sheet sensor provided below the target patient on a bed, and the sheet sensor detects a generated body pressure signal as the signal.

7. An evaluation method for evaluating the severity of pneumonia in a target patient, comprising:
an acquisition step of acquiring a respiratory waveform of the target patient;
a calculation step of calculating a value of an index indicating instability of a respiratory cycle or a respiratory frequency from the respiratory waveform; and
an evaluation step of evaluating the severity based on the calculated value.

8. An evaluation program for causing a computer to operate as each unit of the evaluation device according to any one of claims 1 to 4.

9. A computer-readable recording medium in which the evaluation program according to claim 8 is recorded.
